# EUROPÄISCHE PATENTANMELDUNG

(11) **EP 0 624 338 A2**
(43) Veröffentlichungstag der Anmeldung: **17.11.1994**
(21) Anmeldenummer: 94107032.8
(22) Anmeldetag: 05.05.1994
(51) Int. Cl.: A61B 5/0408

(54) **Körperelektrode**

(30) Priorität: 13.05.1993 DE 4315987
(71) Anmelder: ARBO-tec Sensor-Technologie GmbH, D-38820 Halberstadt (DE)
(72) Erfinder: Barth, Heinz-Günter, D-38820 Halberstadt (DE); Krüger, Rainer, D-38820 Halberstadt (DE); Lüddecke, Ingolf, D-38820 Halberstadt (DE)
(74) Vertreter: Rehmann, Klaus-Thorsten, Dipl.-Ing.

(57) **Zusammenfassung**

Bei einer Körperelektrode mit einer Trägerschicht (1) bestehend aus einem Elektrodenteil (2), auf das ein elektrisch leitender Sensor (4) aufgebracht ist, und einem als Anschlußteil dienenden Leitungsteil (3), das eine elektrisch leitende, mit dem leitenden Sensor (4) verbundene Schicht (11, 12, 13) aufweist, und mit einer die freie Fläche des Sensors (4) abdeckenden, für den Hautkontakt vorgesehenen klebenden und leitenden Schicht (17), wird die Störanfälligkeit der aufgenommenen Körpersignale dadurch herabgesetzt, daß zwischen dem Sensor (4) und dem Leitungsteil (3) in einer fensterartigen Ausnehmung (9) der Trägerschicht (1) eine aktive elektrische Schaltung (10) eingesetzt ist und daß das Leitungsteil (3) mit Versorgungsleitungen (12, 13) zur Zuführung der Betriebsspannung (U_{B}, Masse) für die aktive elektrische Schaltung und wenigstens einer Signalleitung (11) versehen ist. Dadurch ist es möglich, die Anschlußleitung ohne aufwendige Maßnahmen zur Verringerung des Einflusses von Störungen auszubilden. Bevorzugt ist eine Ausbildung der Anschlußleitung mit einem einstückig mit dem Elektrodenteil (2) verbundenen Leitungsteil (3). Als Trägerschicht (1) kommen insbesondere Kunststoffolien und Papier in Frage.

## Beschreibung

Die Erfindung betrifft eine Körperelektrode mit einer Trägerschicht, bestehend aus einem Elektrodenteil, auf das ein elektrisch leitender Sensor aufgebracht ist, und einem Anschlußteil, das eine elektrisch leitende, mit dem leitenden Sensor verbundene Schicht aufweist, und mit einer die freie Fläche des Sensors abdeckenden, für den Hautkontakt vorgesehenen klebenden und leitenden Schicht.

Körperelektroden weisen in herkömmlicher Bauart eine leitende und klebende Schicht auf, die den Hautkontakt herstellt. In diese Schicht ragt regelmäßig ein metallischer Sensor, überwiegend aus Ag/AgCl, hinein. An den Sensor ist ein Anschlußstück angeschweißt, angelötet oder angeklebt, das mit einem Anschlußkabel verbunden ist. Die Sensoranordnung und die klebende und leitende Schicht ist mit einer meist formstabileren Abdeckschicht geschützt.

Von der Ludlow Corporation ist unter der Bezeichnung "Perfect Circuit" eine Körperelektrode vorgestellt worden, die in der Technik von gedruckten Schaltungen hergestellt ist. Auf einer glasklar durchsichtigen Trägerschicht ist der Sensor in Form einer gedruckten Schaltung aufgebracht. Die Trägerschicht weist einseitig einen sich nur geringfügig konisch verschmalernden Ansatz auf, auf den vollflächig eine Anschlußfläche einstückig mit dem Sensor als gedruckte Schaltung aufgebracht ist. Die Sensoranordnung ist ausschließlich der Anschlußfläche mit einer klebenden und leitenden Schicht abgedeckt. Die Gesamtanordnung ist auf eine Schutzfolie aufgeklebt und kann zum Gebrauch von dieser abgezogen werden. Die so hergestellte Elektrode weist eine minimale Bauhöhe auf und ist einfach herzustellen. In ihrem Gebrauch stellen sich jedoch Nachteile heraus. Diese bestehen insbesondere darin, daß die Kunststoffträgerschicht einen hohen Biegewiderstand aufweist und somit für die Herstellung eines elektrischen Kontaktes, beispielsweise mit einer Klemmverbindung, erforderliche Verbiegungen der Anschlußfläche auf das Elektrodenteil unmittelbar übertragen werden, wodurch es zu partiellen Ablösungen der leitenden und klebenden Schicht von der Haut kommen kann, so daß Artefakte auftreten. Die Klemmverbindung and der Anschlußfläche des Elektrodenteils schränkt die Anwendbarkeit der Elektrode ein, weil der Patient wegen der Klemmverbindung beispielsweise nicht oder nur unter großer Behinderung auf der Elektrode liegen kann.

Die US-PS 4,539,995 offenbart eine Körperelektrode, die aus einem Elektrodenteil, auf das ein elektrisch leitender Sensor aufgebracht ist, und einem als Anschlußteil dienenden Leitungsteil, das mehrere an den Sensor angeschlossene Leiter aufweist, die parallel übereinander geführt und voneinander isoliert und abgeschirmt sind. Durch die aufwendig Abschirmung der Leitungen sollen Störeinflüsse auf schwache oder schlecht abgeleitete Körpersignale auf ihrem Weg zur Auswertungseinrichtung minimiert werden. Durch den großen Querschnitt weist das Leitungsteil aber zwangsweise einen hohen Biegewiderstand auf, so daß erforderliche Verbiegungen bei der Herstellung des elektrischen Kontakts mit einer Klemmverbindung unmittelbar auf das Elektrodenteil übertragen werden, so daß die oben genannten Nachteile auch hier gelten.

In dem Buch von L.A. Geddes, "Electrodes and the Measurement of Bioelectric Events", Wiley-Interscience, 1972, Seite 98 bis 106, ist eine Körperelektrode aus Metall beschrieben, in deren Gehäuse eine Signalverstärkungsschaltung integriert ist. Die elektrische Schaltung ist oberhalb des Sensors in dem zylindrischen Gehäuse, das als elektrostatischer Schutz dient, untergebracht. Derartige Elektroden sind wenig komfortabel zu handhaben.

Die vorliegende Erfindung hat zum Ziel, eine gattungsgemäße Körperelektrode so fortzubilden, daß zum einen Manipulationen an dem Anschlußteil bei der Herstellung des elektrischen Kontakts keine Auswirkungen auf den Sensorteil haben und zum anderen die sensierten Körpersignale mit einer geringen Störanfälligkeit übertragen werden und dennoch die Handhabung der Körperelektrode und der Komfort für den Patienten hoch sind.

Erfindungsgemäß ist die Körperelektrode der eingangs erwähnten Art dadurch gekennzeichnet, daß zwischen dem Sensor und dem Leitungsteil in einer fensterartigen Ausnehmung der Trägerschicht eine aktive elektrische Schaltung eingesetzt ist und daß das Leitungsteil mit Versorgungsleitungen zur Zuführung der Betriebsspannung für die aktive elektrische Schaltung und wenigstens einer Signalleitung versehen ist.

Die erfindungsgemäße Körperelektrode ist mit einer auf der Trägerschicht der Körperelektrode getragenen und mit dem leitenden Sensor sowie der leitenden Schicht des Anschlußteils verbundenen aktiven elektrischen Schaltung versehen, die als integriertes Schaltungsplättchen sehr klein ausgeführt sein kann und daher unproblematisch auf einer erfindungsgemäßen, auf einer Trägerschicht realisierten Elektrode integriert werden kann. Durch die aktive elektrische Schaltung können die durch den Sensor aufgenommenen Körpersignale unmittelbar im Anschluß an den Sensor verstärkt werden, so daß sie für Störeinflüsse in einem wesentlich geringeren Umfang anfällig sind und daher keine aufwendigen Leitungen für die Ableitung der Körpersignale benötigt werden.

In einer besonders bevorzugten Ausführungsform der Erfindung ist es daher möglich, daß die an die aktive elektrische Schaltung angeschlossene Signalleitung auf das Leitungsteil aufgebracht und auf der der Trägerschicht abgewandten Seite mit einer isolierenden Schicht abgedeckt und so allseitig isoliert ist. In dieser Ausführungsform der erfindungsgemäßen Körperelektrode ist die Anschlußleitung einstückig in sie integriert. Dies hat zur Folge, daß Sensor und Anschlußleitung im selben Verfahrensschritt gebildet sein können. Die Integration der Anschlußleitung in die Elektrode hat ferner den Vorteil, daß bereits aufgrund der Formgebung eine weitgehende mechanische Entkopplung zwischen dem Anschlußende und dem Elektrodenteil entsteht. Diese aufgrund der Formgebung bestehende mechanische Entkopplung wird noch dadurch verbessert, daß eine sehr flexible Trägerschicht verwendet wird, die dafür sorgt, daß sich am Ende des Leitungsteils auftretende Biege- und Drehmomente nicht merklich auf das Elektrodenteil übertragen. Da die Kontaktierung zwischen der Signalleitung und beispielsweise einer mehrpoligen Weiche aufgrund des integrierten Leitungsteils patientenfern erfolgt, entfällt eine Störung des Patienten durch das Verbindungselement, beispielsweise Klemmelement, mit dem die Kontaktierung hergestellt wird.

Vorzugsweise sind auch die Versorgungsleitungen auf der Trägerschicht neben der Signalleitung aufgebracht. Dies hat zur Folge, daß die Kontaktierung für die Versorgungsspannung ebenfalls patientenfern erfolgt.

Durch die Anordnung von Versorgungsleitung und Signalleitung auf derselben Schicht nebeneinander entsteht der Vorteil, daß die hohe Flexibilität unproblematisch gewahrt bleibt.

Es ist jedoch auch möglich, die Versorgungsleitungen auf einer eigenen Trägerschicht, also einer von der Trägerschicht der Signalleitung verschiedenen Trägerschicht, des Anschlußteils aufzubringen und mit einer isolierenden Schicht abzudecken. Durch den mehrschichtigen Aufbau wird die Trägerschicht etwas stabiler, kann jedoch auch etwas schmaler ausgebildet sein, so daß die angestrebte geringe Torsionssteifigkeit auch in dieser Ausführungsform erzielbar ist.

Es ist zweckmäßig, wenn die abdeckende Schicht mit der Trägerschicht beiderseits der Signalleitung vollflächig verbunden ist. Dies gilt naturgemäß auch, wenn die Versorgungsleitungen neben der Signalleitung auf derselben Trägerschicht angeordnet sind. In diesem Fall ist es wichtig, daß die abdeckende Schicht mit der Trägerschicht vollflächig zwischen und neben den Leitungen verbunden ist.

Trägerschicht und abdeckende Schicht können vorzugsweise aus demselben Material, z.B. Kunststoff gebildet sein. Die durchsichtige, vorzugsweise glasklare, Ausbildung der Schichten hat den Vorteil, daß die gedruckten Leitungen bzw. der gedruckte Sensor optisch prüfbar sind, so daß etwaige Leitungsbrüche regelmäßig optisch erkennbar sind. Ferner kann die Haut unter der Elektrode, zumindest außerhalb des aufgedruckten Sensors und der aufgedruckten Leitungen beobachtet werden, während die Elektrode aufgesetzt ist.

Zur Herstellung einer elektrischen Verbindung am Ende des Leitungsteils endet zweckmäßigerweise die abdeckende Schicht zur Ausbildung einer Kontaktfläche mit Abstand von dem freien Ende der Trägerschicht und der Leitungen. Es ist denkbar, an das freie Ende der Signalleitung ein Kabel beispielsweise mit einer Klemmvorrichtung anzuschließen. Dabei ist besonders bevorzugt, eine mehradrige Weiche mit einer entsprechenden Anzahl von Klemmvorrichtungen auszubilden und so mehrere Elektroden mit ihren Signalleitungen an der Weiche zu kontaktieren. Dabei ist es zweckmäßig, wenn das Kontaktende des Leitungsteils verbreitert ist, um ein versehentliches Einstecken in etwaige spannungsführende Teile unmöglich zu machen. Diese Art der Kontaktierung erscheint vorteilhaft gegenüber der ebenfalls denkbaren Kontaktierung des freien Endes der Signalleitung mit einem herkömmlichen Steckkontakt.

Die abdeckende Schicht kann sich vorteilhaft bis in den Elektrodenteil hinein erstrecken und sich dort verbreitern. Hierdurch wird die mechanische Stabilität der Verbindung der Anschlußleitung mit dem Elektrodenteil, insbesondere im Hinblick auf Auszugskräfte, wesentlich verbessert.

Eine optimale Form des Sensors für die erfindungsgemäße Elektrode besteht aus einem Kreisring mit mehreren radialen, sich zentrisch treffenden Stegen. Bei dieser Ausbildung kann sich die abdeckende Schicht bis in den Bereich des äußeren Randes des Kreisringes erstrecken.

In einer bevorzugten Ausführungsform weist das Leitungsteil ein Verhältnis von Länge zu Breite von > 50:1, vorzugsweise > 90:1 auf. In konkreten Ausführungsbeispielen ist die Breite des Leitungsteils zur Breite der aufgedruckten Leitungen so gewählt, daß beiderseits der Leitungen ein Streifen von etwa 0,5 mm existiert, auf dem die Trägerschicht unmittelbar mit der abdeckenden Schicht, flächig verbunden ist, so daß sich eine allseitige Isolierung der Anschlußleitung ergibt. Die Länge des Anschlußteils ergibt sich aus dem jeweiligen Anwendungszweck und sollte regelmäßig > 20 cm sein. In der Praxis werden Längen von 23 bis 60 cm vorgesehen.

Die erfindungsgemäße Körperelektrode läßt sich mit einer Trägerschicht und einer abdeckenden Schicht aus Kunststoff ohne Probleme herstellen. Eine bevorzugte Alternative besteht jedoch darin, Papier als Trägerschicht zu verwenden und ggf. die abdeckende Schicht durch einen aufgebrachten Isolierlack zu realisieren.

Im Unterschied zu der Technik von gedruckten Leitungen wird bei der erfindungsgemäßen Köperelektrode der Sensor und die Anschlußleitung vorzugsweise in einem echten Druckverfahren, insbesondere Siebdruckverfahren, auf die Trägerschicht aufgebracht. Sensor und Leitungen können dabei aus einem leitfähigen Lack bestehen, der vorzugsweise silberhaltig sein kann und vorzugsweise jedenfalls an seiner Oberfläche chloriert ist, um ein optimales Sensorsystem zu gewährleisten.

Die Erfindung wird im folgenden anhand eines in der Zeichnung dargestellten Ausführungsbeispiels näher erläutert. Es zeigen:
- Figur 1 -: eine Draufsicht auf eine erfindungsgemäße Elektrode
- Figur 2 -: einen Längsschnitt durch die Elektrode gemäß Figur 1.

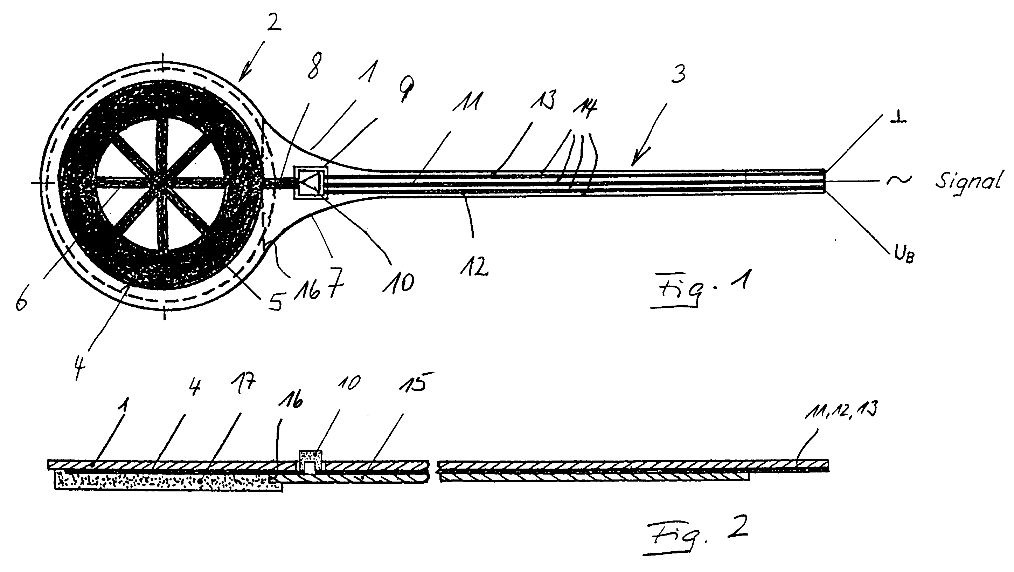

Figur 1 läßt eine Trägerschicht 1 erkennen, die aus einem Elektrodenteil 2 und einem Leitungsteil 3 besteht. Das Elektrodenteil 2 ist im wesentlichen kreisförmig ausgebildet, das Leitungsteil 3 länglich mit einer wesentlich größeren Länge als Breite. Auf das Elektrodenteil 2 ist ein Sensor 4 im Siebdruckverfahren aufgedruck, der aus einem Kreisring 5 und acht speicherähnlichen Stegen 6 besteht, die radial ausgerichtet sind und sich im Zentrum des Kreisrings treffen. Zwischen dem Elektrodenteil 2 und dem Leitungsteil 3 ist die Trägerschicht 1 mit einem Übergangsteil 7 ausgebildet, das einen allmählichen Übergang von einer größeren Breite auf die schmale Breite des Leitungsteils 3 herstellt, sich also konisch verjüngt.

Mit dem Sensor 4 ist einstückig ein Anschlußleitungsstück 8 verbunden, das im Übergangsstück 7 endet. Das Ende des Anschlußleitungsstücks 8 ragt in eine fensterartige Ausnehmung 9 in der Trägerschicht 1 hinein, in die ein aktiver elektrischer Schaltkreis 10 eingesetzt ist. Dieser ist einseitig mit dem Anschlußleitungsstück 8 und auf der anderen Seite mit drei Leitungen 11, 12, 13 verbunden, von denen die mittlere eine Signalleitung 11 und die beiden äußeren Versorgungsleitungen 12, 13 zur Zuführung einer Gleichspannung zum aktiven elektrischen Schaltkreis 10 sind. Die erste Versorgungsleitung 12 ist mit einer Versorgungsspannung U_{B} und die zweite Versorgungsleitung 13 mit Masse verbunden.

Die Leitungen 11, 12, 13 weisen zwischen sich und zum Rand der Trägerschicht 1 hin jeweils einen Streifen 14 auf, über die die Trägerschicht 1 flächig mit einer auf der anderen Seite (Unterseite) der (auf die Unterseite der Trägerschicht 1) aufgedruckten Leitungen 11, 12, 13 aufgebrachten isolierenden Schicht 15 flächig verbunden ist. Die flächige Verbindung ist isolierend ausgeführt, so daß die Leitungen 11, 12, 13 allseitig isoliert sind. Die abdeckende isolierende Schicht 15 erstreckt sich in den Elektrodenteil 2 der Trägerschicht 1 hinein und endet mit einer vorderen Kante 16 im Bereich des äußeren Randes des Ringes 5 des Sensors 4. Im Bereich des Elektrodenteils 2 und des Übergangsteils 7 ist die abdeckende Schicht 15 entsprechend der Form der Trägerschicht 1 verbreitert, wodurch an dem Leitungsteil 3 wirkende Auszugskräfte verbessert aufgenommen werden.

Im Bereich des Elektrodenteils 2 ist der Sensor 4 auf seiner der Trägerschicht 1 abgewandten Seite durch eine klebende und leitende Schicht 17 abgedeckt. Diese haftet an der Trägerschicht 1 und an der metallischen Schicht des Sensors 4. Zur Sicherstellung eines Schutzes vor Kurzschlüssen erstreckt sich die leitende und klebende Schicht 17 bis über den Rand 16 der isolierenden Schicht 15.

Am freien Ende des Leitungsteils 3 endet die isolierende Schicht 15 mit Abstand von dem Ende der Trägerschicht 1 und den Leitungen 11, 12, 13, um die Leitungen 11, 12, 13 einseitig freizulassen. Ein elektrischer Kontakt zu einem weiterführenden Kabel oder einem Anschlußstecker kann in an sich bekannter Weise, vorzugsweise durch eine Klemmverbindung, hergestellt werden.

## Patentansprüche

1. Körperelektrode mit einer Trägerschicht (1) bestehend aus einem Elektrodenteil (2), auf das ein elektrisch leitender Sensor (4) aufgebracht ist, und einem als Anschlußteil dienenden Leitungsteil (3), das eine elektrisch leitende, mit dem leitenden Sensor (4) verbundene Schicht (11, 12, 13) aufweist, und mit einer die freie Fläche des Sensors (4) abdeckenden, für den Hautkontakt vorgesehenen klebenden und leitenden Schicht (17), **dadurch gekennzeichnet, daß** zwischen dem Sensor (4) und dem Leitungsteil (3) in einer fensterartigen Ausnehmung (9) der Trägerschicht (1) eine aktive elektrische Schaltung (10) eingesetzt ist und daß das Leitungsteil (3) mit Versorgungsleitungen (12, 13) zur Zuführung der Betriebsspannung (U_{B}, Masse) für die aktive elektrische Schaltung und wenigstens einer Signalleitung (11) versehen ist.

2. Körperelektrode nach Anspruch 1, **dadurch gekennzeichnet, daß** das Leitungsteil (3) wenigstens einige Zentimeter lang und höchstens wenige Millimeter breit ist, auf das Leitungsteil (3) die an die aktive elektrische Schaltung (10) angeschlossene Signalleitung (11) aufgebracht und auf der der Trägerschicht (1) abgewandten Seite mit einer isolierenden Schicht (15) abgedeckt und so allseitig isoliert ist.

3. Körperelektrode nach Anspruch 2, dadurch gekennzeichnet, daß die Versorgungsleitungen (12, 13) auf der Trägerschicht (1) neben der Signalleitung (11) aufgebracht sind.

4. Körperelektrode nach Anspruch 2, dadurch gekennzeichnet, daß die Versorgungsleitungen (12, 13) auf einer eigenen Trägerschicht des Leitungsteils (3) aufgebracht und mit einer isolierenden Schicht abgedeckt sind.

5. Körperelektrode nach einem der Ansprüche 2 bis 4, dadurch gekennzeichnet, daß die abdeckende Schicht (15) mit der Trägerschicht (1) beiderseits der Leitungen (11, 12, 13) vollflächig verbunden ist.

6. Körperelektrode nach einem der Ansprüche 2 bis 5, dadurch gekennzeichnet, daß die abdeckende Schicht (15) und/oder die Trägerschicht (1) durchsichtig ist.

7. Körperelektrode nach einem der Ansprüche 2 bis 6, dadurch gekennzeichnet, daß die abdeckende Schicht (15) aus dem gleichen Material gebildet ist wie die Trägerschicht (1).

8. Körperelektrode nach einem der Ansprüche 2 bis 7, dadurch gekennzeichnet, daß der Leitungsteil (3) eine solche Flexibilität aufweist, daß sich an seinem Ende wirkende Biege- und Drehmomente nicht merklich auf das Elektrodenteil (2) übertragen.

9. Körperelektrode nach einem der Ansprüche 2 bis 8, dadurch gekennzeichnet, daß die Leitungen (11, 12, 13) des Leitungsteils (3) und der Sensor (4) im gleichen Verfahrensschritt hergestellt sind.

10. Körperelektrode nach einem der Ansprüche 2 bis 9, dadurch gekennzeichnet, daß die abdeckende Schicht zur Ausbildung von Kontaktflächen mit Abstand von dem freien Ende der Trägerschicht (1) und der wenigstens einen Leitung (11, 12, 13) endet.

11. Körperelektrode nach einem der Ansprüche 2 bis 10, dadurch gekennzeichnet, daß sich die abdeckende Schicht (15) bis in den Elektrodenteil (2) hinein erstreckt und sich dort verbreitert.

12. Körperelektrode nach einem der Ansprüche 1 bis 11, dadurch gekennzeichnet, daß der Sensor (4) in Form eines Kreisringes (5) mit mehreren radialen, sich zentrisch treffenden Stegen (6) ausgebildet ist.

13. Körperelektrode nach Anspruch 11 und 12, dadurch gekennzeichnet, daß sich die abdeckende Schicht (15) bis in den Bereich des äußeren Randes des Kreisringes (5) erstreckt.

14. Körperelektrode nach einem der Ansprüche 1 bis 13, dadurch gekennzeichnet, daß das Verhältnis von Länge zu Breite des Leitungsteils (3) > 50:1, vorzugsweise > 90:1 ist.

15. Körperelektrode nach einem der Ansprüche 1-13, dadurch gekennzeichnet, daß das Leitungsteil (3) eine Breite von maximal5 mm und eine Länge von wenigstens 20 cm aufweist.

16. Körperelektrode nach einem der Ansprüche 1 bis 15, dadurch gekennzeichnet, daß die Trägerschicht (1) und/oder die abdeckende Schicht (15) aus Kunststoff bestehen.

17. Körperelektrode nach einem der Ansprüche 1 bis 16, dadurch gekennzeichnet, daß die Trägerschicht (1) aus Papier gebildet ist.

18. Körperelektrode nach einem der Ansprüche 1 bis 17, dadurch gekennzeichnet, daß die abdeckende Schicht (15) aus einem Isolierlack besteht.

19. Körperelektrode nach einem der Ansprüche 2 bis 18, dadurch gekennzeichnet, daß der Sensor (4) und die wenigstens eine Leitung (11, 12, 13) auf dem Leitungsteil (3) mit einem Siebdruckauftrag auf die Trägerschicht (1) aufgebracht sind.

20. Körperelektrode nach Anspruch 19, dadurch gekennzeichnet, daß der Sensor (4) und die wenigstens eine Leitung (11, 12, 13) aus einem leitfähigen Lack bestehen.

21. Körperelektrode nach Anspruch 20, dadurch gekennzeichnet, daß der leitfähige Lack silberhaltig und vorzugsweise an seiner Oberfläche koloriert ist.

22. Körperelektrode nach einem der Ansprüche 1 bis 21, dadurch gekennzeichnet, daß der aktive elektrische Schaltkreis in Form eines integrierten Schaltungsplättchens ausgebildet und mit einer isolierenden flüssig oder pastös aufgetragenen, ausgehärteten Masse abgedeckt ist.
